# EUROPEAN PATENT APPLICATION

(11) **EP 1 593 400 A1**
(43) Date of publication of application: **09.11.2005**
(21) Application number: 05252559.9
(22) Date of filing: 25.04.2005
(51) Int. Cl.: A61L 27/36, A61F 2/02

(54) **Hybrid biologic-synthetic bioabsorbable scaffolds**

(30) Priority: 04.05.2004 US 567886 P
(71) Applicant: Depuy Products, Inc., Warsaw, Indiana 46581 (US)
(72) Inventor: Malaviya, Prasanna, Fort Wayne, IN 46804 (US); Orban, Janine M., Warsaw, IN 46580 (US); Jenks, Philip J., Warsaw, IN 46580 (US); Whalen, Terrence, Leesburg, IN 465838 (US)
(74) Representative: Belcher, Simon James

(57) **Abstract**

A method of making a bioprosthetic device, the method comprises coating a synthetic layer with a comminuted naturally occurring extracellular matrix material, positioning the coated synthetic layer between a first layer of naturally occurring extracellular matrix material and a second layer of naturally occurring extracellular matrix material to make an assembly, and applying positive pressure to the assembly. The synthetic material is a bioabsorbable fibrous material, selected from the group consisting of PLA, PGA, PCL, PDO, TMC, PVA, copolymers thereof, and blends thereof.

## Description

The present invention relates to bioprosthetics and particularly to the use of bioprosthetics for the repair and replacement of connective tissue. More particularly, the present invention relates to the use of a composite bioprosthetic device made up of a synthetic portion and heterologous animal tissue.

Documents which describe the characteristics and properties of small intestine submucosa (SIS) include, US-4352463, US-4902508, US-4956179, US-5281422, US-5372821, US-5445833, US-5516533, US-5573784, US-5641518, US-5645860, US-5668288, US-5695998, US-5711969, US-5730933, US-5733868, US-5753267, US-5755791, US-5762966, US-5788625, US-5866414, US-5885619, US-5922028, US-6056777 and WO-97/37613. SIS, in various forms, is commercially available from Cook Biotech Incorporated (Bloomington, IN). US-4400833 and WO-00/16822 disclose information related to bioprosthetics.

It is also known to use naturally occurring extracellular matrices (ECMs) to provide a scaffold for tissue repair and regeneration. One such ECM is small intestine submucosa (SIS). SIS has been used to repair, support, and stabilize a wide variety of anatomical defects and traumatic injuries. Commercially-available SIS material is derived from porcine small intestinal submucosa that remodels the qualities of its host when implanted in human soft tissues. Further, it is taught that the SIS material provides a natural matrix with a three-dimensional microstructure and biochemical composition that facilitates host cell proliferation and supports tissue remodelling. SIS products, such as the materials sold under the trade marks Oasis and Surgisis are commercially available from Cook Biotech, Bloomington, IN.

An SIS orthobiologic implant product which is sold under the trade mark RESTORE is available from DePuy Orthopaedics, Inc. in Warsaw, Indiana. The DePuy product is described for use during rotator cuff surgery, and is provided as a resorbable framework that allows the rotator cuff tendon to regenerate itself. The DePuy implant is derived from porcine small intestine submucosa that has been cleaned, disinfected, and sterilized. Small intestine submucosa (SIS) has been described as a naturally-occurring ECM composed primarily of collagenous proteins. Other biological molecules, such as growth factors, glycosaminoglycans, etc., have also been identified in SIS, as discussed in Hodde et al., Tissue Eng. 2(3): 209-217 (1996); Voytik-Harbin et al., J. Cell Biochem., 67:478-491 (1997); McPherson and Badylak, Tissue Eng., 4(1): 75-83 (1998); Hodde et al., Endothelium, 8(1):11-24 (2001); Hodde and Hiles, Wounds, 13(5): 195-201 (2001); Hurst and Bonner, J. Biomater. Sci. Polym. Ed., 12(11) 1267-1279 (2001); Hodde et al., Biomaterial, 23(8): 1841-1848 (2002); and Hodde, Tissue Eng., 8(2): 295-308 (2002), all of which are incorporated by reference herein. During seven years of preclinical testing in animals, there were no incidences of infection transmission from the implant to the host, and the SIS material has not decreased the systemic activity of the immune system. See Allman et al., Transplant, 17(11): 1631-1640 (2001); Allman et al., Tissue Eng., 8(1): 53-62 (2002).

While small intestine submucosa is available, other sources of submucosa are known to be effective for tissue remodelling. These sources include, but are not limited to, stomach, bladder, alimentary, respiratory, or genital submucosa, or liver basement membrane, as discussed in US-6379710, US-6171344, US-6099567 and US-5554389. Further, while SIS is most often porcine derived, it is known that these various submucosa materials may be derived from non-porcine sources, including bovine and ovine sources. Additionally, the ECM material may also include partial layers of laminar muscular is mucosa, muscular is mucosa, lamina propria, stratum compactum and/or other tissue materials depending upon factors such as the source from which the ECM material was derived and the delamination procedure.

For the purposes of this invention, it is within the definition of a naturally occurring ECM to clean, delaminate, and/or comminute the ECM, or even to cross-link the collagen fibres within the ECM. It is also within the definition of naturally occurring ECM to fully or partially remove one or more sub-components of the naturally occurring ECM. However, it is not within the definition of a naturally occurring ECM to separate and purify the natural collagen or other components or sub-components of the ECM and reform a matrix material from the purified natural collagen or other components or sub-components of the ECM. While reference is made to SIS, it is understood that other naturally occurring ECMs (e.g., stomach, bladder, alimentary, respiratory, and genital submucosa, and liver basement membrane), whatever the source (e.g., bovine, porcine, ovine) may be used. Thus, in this application, the terms "naturally occurring extracellular matrix" or "naturally occurring ECM" are intended to refer to extracellular matrix material that has been cleaned, disinfected, sterilized, and optionally cross-linked. The terms "naturally occurring extracellular matrix" and "naturally occurring ECM" are also intended to include ECM foam material prepared as described in EP-A-1425024.

There are currently many ways in which various types of tissues such as ligaments and tendons, for example, are reinforced and/or reconstructed. Suturing the torn or ruptured ends of the tissue is one method of attempting to restore function to the injured tissue. Sutures may also be reinforced through the use of synthetic non-bioabsorbable or bioabsorbable materials. Autografting, where tissue is taken from another site on the patient's body, is another means of soft tissue reconstruction. Yet another means of repair or reconstruction can be achieved through allografting, where tissue from a donor of the same species is used. Still another means of repair or reconstruction of soft tissue is through xenografting in which tissue from a donor of a different species is used.

According to the present invention, a bioprosthetic device for soft tissue attachment, reinforcement, and/or reconstruction is provided. The bioprosthetic device comprises SIS or other ECM formed to include a tissue layer, and a synthetic portion coupled to the tissue layer. The tissue layer may also be dehydrated.

In one embodiment, the SIS portion of the bioprosthetic device includes a top tissue layer of SIS material and a bottom tissue layer of SIS material coupled to the top tissue layer. The synthetic portion of the bioprosthetic device includes a row of fibres positioned to lie between the top and bottom tissue layers of the SIS portion. The fibres are positioned to lie in a spaced-apart coplanar relation to one another along a length, L, of the SIS portion. The fibres are each formed to include a length L2, where L2 is longer than L so that an outer end portion of each fibre extends beyond the SIS portion in order to anchor the bioprosthetic device to the surrounding soft tissue.

The synthetic reinforcing portion of the bioprosthetic device may include a mesh member formed to define the same length, L, as the SIS portion, or may include a mesh member having a body portion coupled to the SIS portion and outer wing members coupled to the body portion and positioned to extend beyond the length, L, and a width, W, of the SIS portion in order to provide more material for anchoring the bioprosthetic device to the surrounding soft tissue.

The synthetic reinforcing portion of the device enhances the mechanical integrity of the construct in one (for fibre reinforcements) or two (for fibre or mesh reinforcements) dimensions. For the repair of tissues such as meniscal or articular cartilage, or discs, integrity in three dimensions is desirable for the implant to withstand the shear forces that will be present after implantation. Thus, in one embodiment of the present application, the absorbable synthetic portion of the device is in a three-dimensional form, to provide mechanical strength in three dimensions. The absorbable synthetic may be a fibrous non-woven construct or a three-dimensional woven mesh, for example.

For the repair of certain other types of tissues such as tendons, ligaments, or fascia, tissue infiltration and repair in three dimensions is desirable, although three-dimensional enhanced mechanical integrity of the implant is not necessary. Thus, another embodiment of this invention is a composite device comprised of an SIS portion and an absorbable synthetic foam. The absorbable synthetic foam, in one example, is made of a biocompatible polymer that has a degradation profile that exceeds that of the SIS portion of the device. In this case, the SIS portion of the device provides the initial suturability of the product, and the synthetic foam provides an increased surface area in three dimensions for enhanced tissue infiltration. In a further embodiment, that synthetic foam is made of 65/35 polyglycolic acid/ polycaprolactone, or 60/40 polylactic acid/polycaprolactone, or a 50:50 mix of the two.

The ECM portion of the composite may be provided as a single, hydrated sheet of SIS. Alternatively, the single sheet of SIS is lyophilized (freeze-dried). Such a treatment renders increased porosity to the SIS sheet, thereby enhancing it's capacity for allowing tissue ingrowth. Additionally, this SIS portion may comprise multiple sheets of SIS that have been laminated together by mechanical pressure while hydrated. The laminated SIS assembly optionally further physically crosslinked by partially or fully drying (down to less than 15% moisture content) under vacuum pressure. Alternatively, the laminated SIS assembly is lyophilized, instead of being vacuum dried, to increase its porosity. In still another embodiment, the SIS sheet or laminate is perforated by mechanical means, to create holes ranging, for example, from 1 mm to 1 cm. Another embodiment uses woven textiles of single or multi-layer SIS strips that have been optionally vacuum dried or lyophilized, to create meshes having different-sized openings. The woven mesh SIS optionally is assembled while the SIS is still hydrated and then the whole assembly vacuum-dried or lyophilized. Such a construct is suturable in the short term, and has the advantage of having a very open structure for tissue ingrowth over time.

The three-dimensional synthetic portion of the device is illustratively provided in the form of a fibrous non-woven or foam material. The synthetic portion of the device preferably has interconnecting pores or voids to facilitate the transport of nutrients and/or invasion of cells into the scaffold. The interconnected voids range in size, for example, from about 20 to 400 µm, preferably 50 to 250 µm, and constitute about 70 to 95% of the total volume of the construct. The range of the void size in the construct can be manipulated by changing process steps during construct fabrication. The foam optionally may be formed around a reinforcing material, for example, a knitted mesh.

The synthetic reinforcing portion of the device is made of a fibrous matrix made, for example, of threads, yarns, nets, laces, felts, and non-wovens. An illustrated method of combining the bioabsorbable fibrous materials, e.g. fibres, to make the fibrous matrix for use in devices of the present invention is known to one skilled in the art as the wet lay process of forming non-wovens. The wet lay method has been described in "Non-woven Textiles," by Radko Krcma, Textile Trade Press, Manchester, England, 1967 pages 175-176.

Alternatively, the synthetic reinforcing portion of the device is made of a three-dimensional mesh or textile. A preferred method of combining the bioabsorbable fibrous materials, e.g. fibres, to make the fibrous matrix for use in devices of the present invention is known to one skilled in the art as three-dimensional weaving or knitting. The three-dimensional weaving/knitting or braiding method has been described by several groups who have used the constructs for tissue engineering applications including Chen et al. in "Collagen Hybridization with Poly(1-Lactic Acid) Braid Promotes Ligament Cell Migration," Mater. Sci. Eng. C, 17(1-2), 95-99(2001), and Bercovy et al., in "Carbon-PLGA Prostheses for Ligament Reconstruction Experimental Basis and Short Term Results in Man," Clin. Orthop. Relat. Res., (196), 159-68(1985). Such a three-dimensional material can provide both reinforcement and three-dimensional form.

The synthetic reinforcing portion of the tissue implant of the present invention may include textiles with woven, knitted, warped knitted (i.e., lace-like), non-woven, and braided structures. In an exemplary embodiment the reinforcing component has a mesh-like structure. However, in any of the above structures, mechanical properties of the material can be altered by changing the density or texture of the material. The fibres used to make the reinforcing component can be for example, monofilaments, yarns, threads, braids, or bundles of fibres. These fibres can be made of any biocompatible material, including bioabsorbable materials such as polylactic acid (PLA), polyglycolic acid (PGA), polycaprolactone (PCL), polydioxanone (PDO), trimethylene carbonate (TMC), polyvinyl alcohol (PVA), copolymers or blends thereof. In an exemplary embodiment, the fibres that comprise the non-woven or three-dimensional mesh are formed of a polylactic acid and polyglycolic acid copolymer at a 95:5 mole ratio.

The ECM and the synthetic three-dimensional portion are provided in layers. It is understood for the purposes of this invention that the term "coupled to" describes a relationship wherein a surface of one layer is in contact with a surface of another layer and the two surfaces are connected through mechanical or chemical means, such as through lamination, crosslinking, diffusion of the material of one layer into interstices of the adjacent layer, stitching, and the like. "Sandwiched between" describes a relationship wherein a middle layer has a first surface in contact with a surface of an adjacent layer, and a second opposite-facing surface in contact with a surface of a second adjacent layer. Again, it is understood that the sandwiched layers are connected through mechanical or chemical means. The synthetic reinforcing portion may be provided as individual fibres or as layers. The synthetic reinforcing portion may be imbedded within a foam layer, provided between two other layers that are otherwise coupled together, or may form a layer that is coupled to one or more adjacent layers.

The devices of the present invention can be combined with one or more bioactive agents (in addition to those already present in naturally occurring ECM), one or more biologically-derived agents or substances, one or more cell types, one or more biological lubricants, one or more biocompatible inorganic materials, one or more biocompatible synthetic polymers and one or more biopolymers. Moreover, the devices of the present invention can be combined with devices containing such materials.

"Bioactive agents" include one or more of the following: chemotactic agents; therapeutic agents (e.g. antibiotics, steroidal and non-steroidal analgesics and antiinflammatories, anti-rejection agents such as immunosuppressants and anti-cancer drugs); various proteins (e.g. short chain peptides, bone morphogenic proteins, glycoprotein and lipoprotein); cell attachment mediators; biologically active ligands; integrin binding sequence; ligands; various growth and/or differentiation agents (e.g. epidermal growth factor, IGF-I, IGF-II, TGF-β I-III, growth and differentiation factors, vascular endothelial growth factors, fibroblast growth factors, platelet derived growth factors, insulin derived growth factor and transforming growth factors, parathyroid hormone, parathyroid hormone related peptide, bFGF; TGF_{β} superfamily factors; BMP-2; BMP-4; BMP-6; BMP-12; sonic hedghog; GDF5; GDF6; GDF8; PDGF); small molecules that affect the upregulation of specific growth factors; tenascin-C; hyaluronic acid; chondroitin sulphate; fibronectin; decorin; thromboelastin; thrombin-derived peptides; heparin-binding domains; heparin; heparan sulphate; DNA fragments and DNA plasmids. If other such substances have therapeutic value in the orthopaedic field, it is anticipated that at least some of these substances will have use in the present invention, and such substances should be included in the meaning of "bioactive agent" and "bioactive agents" unless expressly limited otherwise.

"Biologically derived agents" include one or more of the following: bone (autograft, allograft, and xenograft) and derivates of bone; cartilage (autograft, allograft, and xenograft), including, for example, meniscal tissue, and derivatives; ligament (autograft, allograft, and xenograft) and derivatives; derivatives of intestinal tissue (autograft, allograft, and xenograft), including for example submucosa; derivatives of stomach tissue (autograft, allograft, and xenograft), including for example submucosa; derivatives of bladder tissue (autograft, allograft, and xenograft), including for example submucosa; derivatives of alimentary tissue (autograft, allograft, and xenograft), including for example submucosa; derivatives of respiratory tissue (autograft, allograft, and xenograft), including for example submucosa; derivatives of genital tissue (autograft, allograft, and xenograft), including for example submucosa; derivatives of liver tissue (autograft, allograft, and xenograft), including for example liver basement membrane; derivatives of skin tissue; platelet rich plasma (PRP), platelet poor plasma, bone marrow aspirate, demineralized bone matrix, insulin derived growth factor, whole blood, fibrin and blood clot. Purified ECM and other collagen sources are also intended to be included within "biologically derived agents." If other such substances have therapeutic value in the orthopaedic field, it is anticipated that at least some of these substances will have use in the present invention, and such substances should be included in the meaning of "biologically-derived agent" and "biologically-derived agents" unless expressly limited otherwise.

"Biologically derived agents" also include bioremodelable collageneous tissue matrices. The expressions "bioremodelable collagenous tissue matrix" and "naturally occurring bioremodelable collageneous tissue matrix" include matrices derived from native tissue selected from the group consisting of skin, artery, vein, pericardium, heart valve, dura mater, ligament, bone, cartilage, bladder, liver, stomach, fascia and intestine, tendon, whatever the source. Although "naturally occurring bioremodelable collageneous tissue matrix" is intended to refer to matrix material that has been cleaned, processed, sterilized, and optionally crosslinked, it is not within the definition of a naturally occurring bioremodelable collageneous tissue matrix to purify the natural fibres and reform a matrix material from purified natural fibres. The term "bioremodelable collageneous tissue matrices" includes "extracellular matrices" within its definition.

"Cells" include one or more of the following: chondrocytes; fibrochondrocytes; osteocytes; osteoblasts; osteoclasts; synoviocytes; bone marrow cells; mesenchymal cells; stromal cells; stem cells; embryonic stem cells; precursor cells derived from adipose tissue; peripheral blood progenitor cells; stem cells isolated from adult tissue; genetically transformed cells; a combination of chondrocytes and other cells; a combination of osteocytes and other cells; a combination of synoviocytes and other cells; a combination of bone marrow cells and other cells; a combination of mesenchymal cells and other cells; a combination of stromal cells and other cells; a combination of stem cells and other cells; a combination of embryonic stem cells and other cells; a combination of precursor cells isolated from adult tissue and other cells; a combination of peripheral blood progenitor cells and other cells; a combination of stem cells isolated from adult tissue and other cells; and a combination of genetically transformed cells and other cells. If other cells are found to have therapeutic value in the orthopaedic field, it is anticipated that at least some of these cells will have use in the present invention, and such cells should be included within the meaning of "cell" and "cells" unless expressly limited otherwise. Illustratively, in one example of embodiments that are to be seeded with living cells such as chondrocytes, a sterilized implant may be subsequently seeded with living cells and packaged in an appropriate medium for the cell type used. For example, a cell culture medium comprising Dulbecco's Modified Eagles Medium (DMEM) can be used with standard additives such as non-essential amino acids, glucose, ascorbic acid, sodium pyrovate, fungicides, antibiotics, etc., in concentrations deemed appropriate for cell type, shipping conditions, etc.

"Biological lubricants" include: hyaluronic acid and its salts, such as sodium hyaluronate; glycosaminoglycans such as dermatan sulphate, heparan sulphate, chondroiton sulphate and keratan sulphate; synovial fluid and components of synovial fluid, including mucinous glycoproteins (e.g. lubricin), tribonectins, articular cartilage superficial zone proteins, surface-active phospholipids, lubricating glycoproteins I, II; vitronectin; and rooster comb hyaluronate. "Biological lubricant" is also intended to include commercial products such as the high molecular weight sodium hyaluronate product sold in Europe by DePuy International Ltd under the trade mark ARTHREASE, which is manufactured by Bio-Technology General (Israel) Ltd., of Rehovot, Israel, the product distributed by Wyeth-Ayerst Pharmaceuticals under the trade mark SYNVISC Hylan G-F 20, which is manufactured by Biomatrix Inc, the sodium hyaluronate product sold by Sanofi-Synthelabo Inc under the trade mark HYLAGAN, which is manufactured by Fidia SpA, and the sodium hyaluronate product sold in concentrations of 1%, 1.4% and 2.3% (for opthalmologic uses) under the trade mark HEALON by Pharmacia Corporation. If other such substances have therapeutic value in the orthopaedic field, it is anticipated that at least some of these substances will have use in the present invention, and such substances should be included in the meaning of "biological lubricant" and "biological lubricants" unless expressly limited otherwise.

"Biocompatible polymers" is intended to include both synthetic polymers and biopolymers (e.g. collagen). Examples of biocompatible polymers include: polyesters of [alpha]-hydroxycarboxylic acids, such as poly(L-lactide) (PLLA) and polyglycolide (PGA); poly-p-dioxanone (PDO); polycaprolactone (PCL); polyvinyl alcohol (PVA); polyethylene oxide (PEO); polymers disclosed in US-6333029 and US-6355699; and other bioresorbable and biocompatible polymer, co-polymer or mixture of polymers or co-polymers that are utilized in the construction of prosthetic implants. In addition, as new biocompatible, bioresorbable materials are developed, it is expected that at least some of them will be useful materials from which orthopaedic devices may be made.

"Biocompatible inorganic materials" include materials such as hydroxyapatite, all calcium phosphates, alpha-tricalcium phosphate, beta-tricalcium phosphate, calcium carbonate, barium carbonate, calcium sulphate, barium sulphate, polymorphs of calcium phosphate, sintered and non-sintered ceramic particles, and combinations of such materials. If other such substances have therapeutic value in the orthopaedic field, it is anticipated that at least some of these substances will have use in the present invention, and such substances should be included in the meaning of "biocompatible inorganic material" and "biocompatible inorganic materials" unless expressly limited otherwise.

It is expected that various combinations of bioactive agents, biologically derived agents, cells, biological lubricants, biocompatible inorganic materials, biocompatible polymers can be used with the devices of the present invention.

Thus, in one aspect of this invention a bioprosthetic device is provided comprising a layer of ECM material having a first surface, and a three-dimensional synthetic portion having a first surface, wherein the first surface of the ECM layer is coupled to the first surface of the three-dimensional synthetic portion. The three-dimensional synthetic portion may be a fibrous material, illustratively selected from the group consisting of mesh, textile, and felt. Alternatively, the three-dimensional synthetic portion may be a synthetic foam.

In another aspect of this invention a prosthetic device is provided comprising one or more layers of bioremodelable collageneous tissue matrices material coupled to one or more three-dimensional synthetic bodies to provide a three-dimensional composite for tissue attachment, reinforcement, or reconstruction.

In a further aspect, the invention provides a method for making a bioprosthetic device is provided, the method comprising the steps of providing a layer of ECM material having a first surface, placing a polymer solution in contact the first surface of the ECM material to make an assembly, wherein the polymer is selected to form a foam upon lyophilization, and lyophilizing the assembly.

Embodiments of the invention will now be described by way of example with reference to the accompanying drawings, in which:

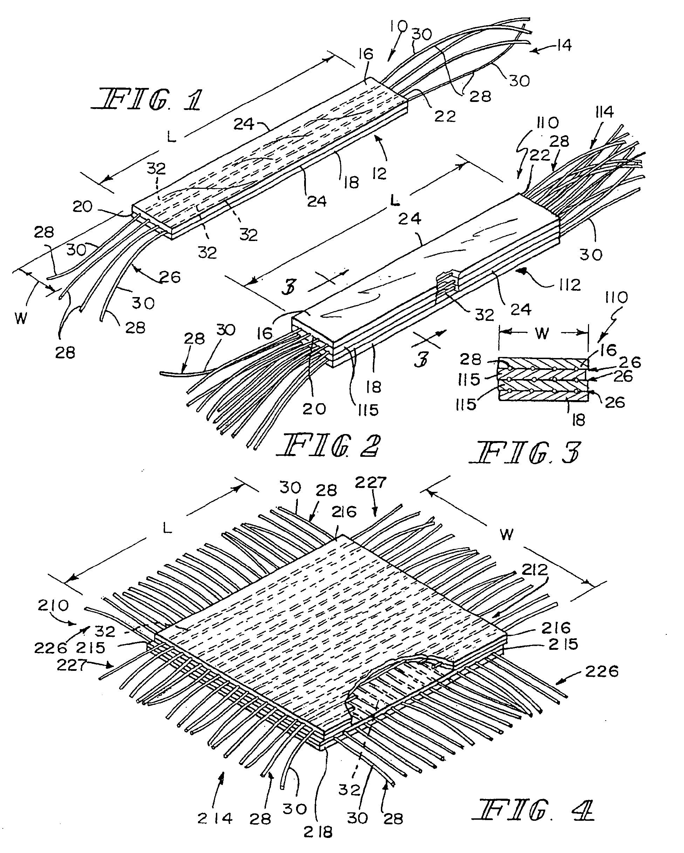

Fig. 1 is a perspective view showing a composite bioprosthetic device of the present invention formed to include a small intestinal submucosa (SIS) portion and a synthetic portion and showing the SIS portion including a top tissue layer of SIS material and a bottom tissue layer of SIS material and further showing the synthetic portion including a row of four fibres positioned to lie in coplanar relation to each other between the top and bottom tissue layers of the SIS portion and positioned to run longitudinally along a length of the SIS portion and extend beyond a first and second end of the SIS portion in order to anchor the bioprosthetic device to surrounding soft tissue;
Fig. 2 is a perspective view similar to Fig. 1 showing an SIS portion of another bioprosthetic device of the present invention being formed to include a top layer, a bottom layer, and two middle layers positioned to lie between the top and the bottom layers and a synthetic device being formed to include three rows of four fibres so that each row is positioned to lie between each of the adjacent tissue layers of the SIS portion so that each fibre is positioned to run longitudinally along a length, L, of the SIS portion;
Fig. 3 is a sectional view taken along line 3-3 of Fig. 2 showing the top, bottom, and middle tissue layers of the SIS portion and also showing the three rows of fibres of the synthetic portion of the bioprosthetic device;
Fig. 4 is a perspective view showing an SIS portion of yet another bioprosthetic device of the present invention being formed to include four tissue layers, similar to Fig. 2, and also showing a synthetic portion of the bioprosthetic device including a first row of multiple fibres positioned to lie between two tissue layers of the SIS portion along a length, L, of the SIS portion and a second row of multiple fibres positioned to lie between two other tissue layers of the SIS portion along a width, W, of the SIS portion;
Fig. 5 is an exploded perspective view of another bioprosthetic device of the present invention showing an SIS portion of the prosthetic device including top, bottom, and middle tissue layers and showing a synthetic portion including a first and a second mesh member positioned to lie between the top and middle tissue layers of and the middle and bottom tissue layers of the SIS portion, respectively;
Fig. 6 is a sectional view of the bioprosthetic device of Fig. 5 showing first and second mesh members "sandwiched" between the tissue layers of the SIS portion of the device;
Fig. 7 is a perspective view showing an SIS portion of another bioprosthetic device being formed to include a top and a bottom tissue layer and further showing a synthetic portion being formed to include a mesh member having a body portion positioned to lie between the top and bottom tissue layers and outer wing portions provided for anchoring the device to surrounding soft tissue;
Fig. 8 is a perspective view showing an SIS portion of yet another bioprosthetic device being formed to include a circularly shaped top and bottom tissue layers each having a diameter, D1, and further showing a synthetic portion of the device being formed to include a circular mesh member positioned to lie between the top and bottom tissue layers and having a diameter, D2, which is larger than D 1 so that an outer rim portion of the mesh member is formed to extend beyond the top and bottom tissue layers for anchoring the bioprosthetic device to the host tissue during surgery;
Fig. 9 is a sectional view of a bioprosthetic device similar to the bioprosthetic device of Fig. 5, having two SIS layers, a reinforcing mesh material between the SIS layers, and a reinforced three-dimensional foam portion adjacent one of the SIS layers;
Fig. 10 is sectional view of another bioprosthetic device, wherein the SIS layer is sandwiched between two foam layers;
Fig. 11 is sectional view of another bioprosthetic device, wherein a foam layer is sandwiched between SIS layers;
Fig. 12 is a sectional view of another bioprosthetic device, wherein a three-dimensional synthetic layer is sandwiched between two SIS layers; and
Fig. 13 is a perspective view showing an SIS portion for use in another bioprosthetic device, wherein the SIS layer is made from weaving strips of SIS.

A composite bioprosthetic device 10, as shown in Fig. 1, is provided for the purposes of soft tissue attachment, reinforcement, and/or reconstruction. Bioprosthetic device 10 includes a small intestinal submucosa (SIS) portion 12 and a synthetic portion 14. SIS portion 12 is provided to be absorbed into the body and replaced by host tissue. SIS portion 12 acts as a scaffold for tissue ingrowth and remodelling. Synthetic portion 14 of bioprosthetic device 10 provides additional initial mechanical strength to bioprosthetic device 10. Because device 10 includes SIS portion 12 and synthetic portion 14, bioprosthetic device 10 is provided with a differential in biodegradation and bioremodelling rates. Synthetic portion 14, for example, can be configured to degrade at a slower rate than SIS portion 12. Further, synthetic portion 14 may act as an anchor to couple bioprosthetic device 10 to the surrounding soft tissue (not shown) during surgery. Alternatively, the SIS portion may be sutured to couple the bioprosthetic device to the surrounding tissue.

SIS portion 12 of bioprosthetic device 10, as shown in Fig. 1, includes a top tissue layer 16 and a bottom tissue layer 18 coupled to top tissue layer 16 mechanically or through a dehydration process. Although top and bottom tissue layers 16, 18 are provided in bioprosthetic device 10 shown in Fig. 1, SIS portions 12 having any number of tissue layers may be used. Perforated tissue layers or any other physical configuration of SIS may also be used, for example as shown in Figs. 2 to 4. Each of the top and bottom tissue layers 16, 18 may comprise multiple tissue layers. In preferred embodiments, for example, top and bottom tissue layers 16, 18 each include three to four layers of SIS tissue. SIS portion 12 further includes a first end 20, a second end 22 spaced-apart from first end 20, and sides 24 coupled to and positioned to lie between first and second ends 20, 22. A length, L, is defined as the distance between first end 20 and second end 22 and a width, W, is defined as the distance between sides 24.

Synthetic portion 14 of bioprosthetic device 10 includes a row 26 of four fibres 28, as shown in Fig. 1. Any fibrous material may be used. Fibres 28 are positioned to lie along length L between top and bottom tissue layers 16, 18 and are further positioned to lie in coplanar relation to one another. When making bioprosthetic device 10, fibres 28 of synthetic portion 14 are placed between top and bottom tissue layers 16, 18 prior to dehydration. Although row 26 of four fibres 28 is provided in bioprosthetic device 10 shown in Fig. 1, synthetic portions 14 formed to include any number of rows 26 having any number of fibres 28 may be used. Fibres 28 made from bioabsorbable and non-bioabsorbable materials may be used. For example, fibres 28 that might be used include those made from polylactic acid (PLA) or polyglycolic (PGA) acid, a combination of the two, the absorbable suture material that is sold by Ethicon Inc under the trade mark Panacryl, and other bioabsorbable materials, such as polyamides, polyethylenes, fluoropolymers (such as polytetrafluoroethylene), aramid polymers, polyesters, carbon fibres and other non-bioabsorbable materials (including those sold under the trade marks Nylon, Kevlar and Dacron).

As shown in Fig. 1, each fibre 28 of bioprosthetic device 10 includes two outer end portions 30 a middle portion 32 coupled to and positioned to lie between outer end portions 30. Middle portion 32 is positioned to lie between top tissue layer 16 and bottom tissue layer 18 of SIS portion 12. Middle portion 32 of fibres 28 helps to provide strength along length, L, of bioprosthetic device 10. One or more outer end portions 30 of fibres 28 can be used for anchoring bioprosthetic device 10 to surrounding soft tissue (not shown). The combination of SIS portion 12 and fibres 28 further provide bioprosthetic device 10 with differential biodegradation rates. For example, fibres 28 of synthetic portion 14 can be made to be non-bioabsorbable or can be made with material which absorbs into the body at a slower rate than SIS portion 12. Uses for bioprosthetic device 10 shown in Fig. 1 include, but are not limited to, ligament or tendon repair.

An alternate bioprosthetic device 110 is shown in Figs. 2 and 3. Bioprosthetic device 110 include an alternate SIS portion 112 of having top tissue layer 16, bottom tissue layer 18, and two middle tissue layers 115. Top, bottom, and middle tissue layers 16, 18, 115 include one or more layers of SIS tissue each. SIS portion 112, similar to SIS portion 12, also includes a first end 20, a second end 22 spaced-apart from first end 20, and sides 24. Bioprosthetic device 110 further includes an alternate synthetic portion 114 having three rows 26 of four fibres 28. One row 26 is positioned to lie between top tissue layer 16 and one of the middle tissue layers 115. Another row 26 is positioned to lie between the two middle tissue layers 115, and the final row 26 of fibres 28 is positioned to lie between another one of the middle tissue layers 115 and bottom tissue layer 16, as shown in Fig. 3. Fibres 28 of bioprosthetic device 110, similar to fibres 28 of bioprosthetic device 10, are positioned to lie along length, L, of SIS portion 112.

Although fibres 28 of bioprosthetic devices 10, 110 are positioned to lie along length, L, of each respective SIS portion 12, 112, the device might include a synthetic portion 214 of an alternate bioprosthetic device 210, as shown in Fig. 4, having multidirectional fibres 28 positioned to lie along a length, L, of an SIS portion 212 and along width, W, of SIS portion 212. Synthetic portion 214 of bioprosthetic device 210 includes a first row 226 having seventeen fibres 28 positioned to lie along length, L, of SIS portion 212. Synthetic portion 214 further includes a second row 227 having eighteen fibres 28 positioned to lie along width, W, of SIS portion 212 so that the fibres 28 of first row 226 and second row 227 are positioned to lie orthogonally with respect to each other. Although rows 226 and 227 are positioned to lie in orthogonal relation to one another, the device might include a synthetic portion 214 having first and second rows 226 and 227 which lie at any angular relation to one another. The device might include rows 226 and 227 each having any number of fibres 28.

Similar to bioprosthetic device 110 shown in Fig. 2, bioprosthetic device 210 includes a top tissue layer 216, a bottom tissue layer 218, and two middle tissue layers 215, positioned to lie between top and bottom tissue layers 216, 218. As mentioned before, top, bottom, and middle tissue layers 216, 218, 215 are each formed to include one or more layers of SIS tissue. Although SIS portion 212 of bioprosthetic device 210 is shown to include four tissue layers, it might have a different number of layers. As shown in Fig. 4, first row 226 is positioned to lie between top tissue layer 216 and one of the two middle tissue layers 215 positioned to lie adjacent to top tissue layer 216. Second row 227 is positioned to lie between the other middle tissue layer 215 and bottom tissue layer 218. The rows 226, 227 can be positioned to lie between any tissue layer of device 210.

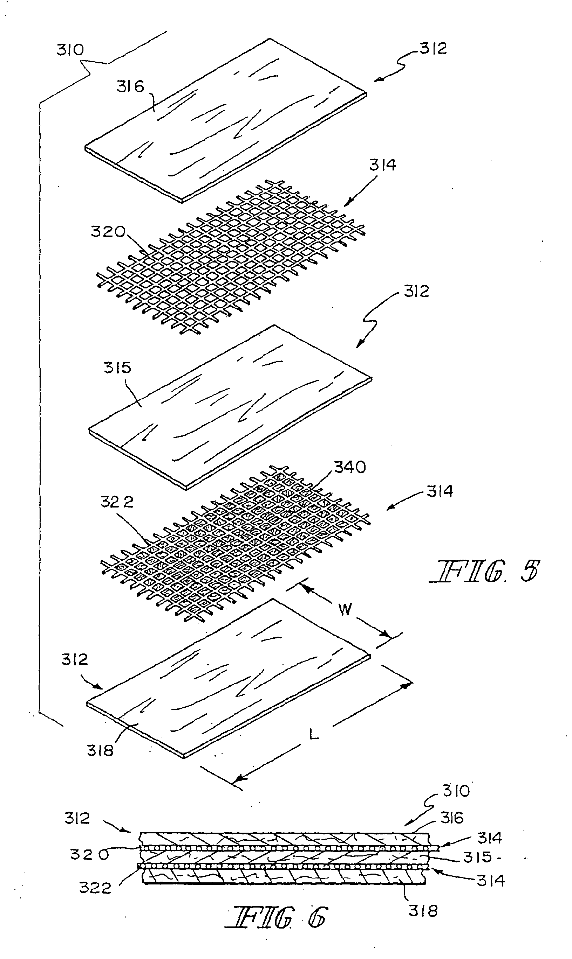

Yet another bioprosthetic device 310 is shown in Figs. 5 and 6. Bioprosthetic device 310 is similar to devices, 10, 110, and 210 and includes an SIS portion 312 having a top tissue layer 316, a bottom tissue layer 318, and a middle tissue layer 315 positioned to lie between top and bottom tissue layers 316, 318. Top, bottom, and middle tissue layers 316, 318, 315 each include one or more layers of SIS tissue. Bioprosthetic device 310 further includes a synthetic portion 314 including first mesh member 320 and second mesh member 322. Other types of synthetic mesh member can be used. For example, bioabsorbable and/or non-bioabsorbable mesh members 320, 322 made of either woven or non-woven PGA and/or PLA mixtures might be used. First mesh member 320 is coupled to and positioned to lie between top tissue layer 316 and middle tissue layer 315 and second mesh member 322 is coupled to and positioned to lie between middle tissue layer 315 and bottom tissue layer 318, as shown in Figs. 5 and 6. Each of the first and second mesh members 320, 322 has a length, L, and a width, W, approximately equal to length, L, and width, W, of tissue layers 315, 316, 318, of SIS portion 312. It is understood that in some embodiments, it may be preferable for the mesh to be slightly smaller than the SIS portion.

In Fig. 5, second mesh member 322 is shown partially coated in comminuted SIS 340. Comminuted SIS may be used to fill the interstices of second mesh member 322 to provide a stronger device. Other means for reinforcing bioprosthetic device 10 may be employed, including suturing or tacking the various layers together. Further, while comminuted SIS is discussed with respect to the embodiment shown in Fig. 5, it is understood that comminuted SIS may be used to coat the mesh or fibres for any embodiment.

Another embodiment of the present invention includes a bioprosthetic device 410 having a synthetic portion 414 including a mesh member 420, as shown in Fig. 7. Similar to the previously mentioned devices, bioprosthetic device 410 includes an SIS portion 412 having a top tissue layer 416 and a bottom tissue layer 418 coupled to top tissue layer 416. Top and bottom tissue layers 416, 418 each include one or more layers of SIS tissue. Mesh member 420 includes a central body portion (not shown) and outer wing portions 430, as shown in Fig. 7. Outer wing portions 430 are extensions of the central body portion. Although four outer wing portions 430 are shown in Fig. 7, the device might include a mesh member having a body portion and any number of wing portions 430 coupled to the body portion. The central body portion of mesh member 420 is formed to include a length and a width equal to length, L, and width, W, of SIS portion 412. The central body portion is coupled to and positioned to lie between top tissue layer 416 and bottom tissue layer 418 of SIS portion 420. Each wing portion 430 is coupled to the central body portion of mesh member 420 and is positioned to extend beyond the length, L, and width, W, of SIS portion 412, as shown in Fig. 7. As mentioned before, outer wing portions 430 are extensions of the central body portion. Wing portions 430 provide additional material for anchoring bioprosthetic device 410 to the surrounding soft tissue. Because outer wing portions 430 extend beyond central body portion of mesh member 420, mesh member 420 has a length and a width greater than length, L, and width, W, of SIS portion 412.

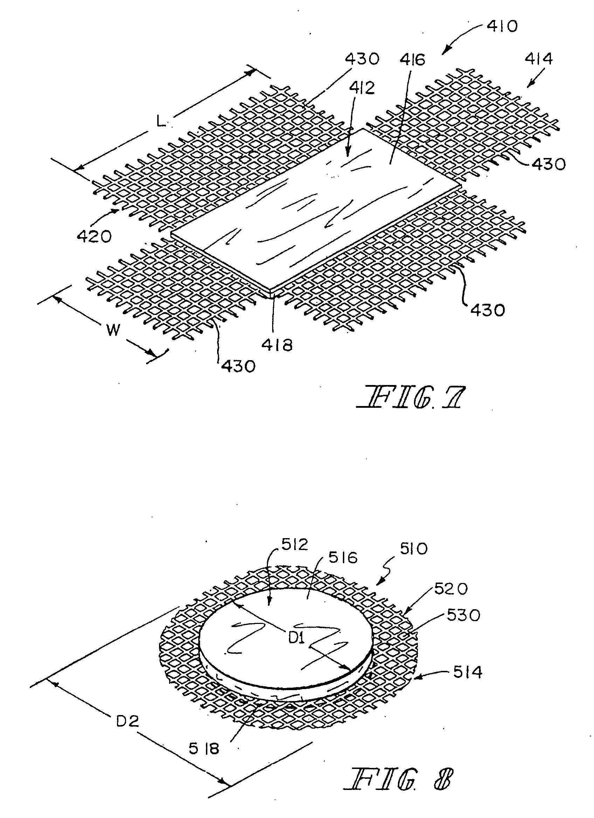

Yet another embodiment of the present invention is shown in Fig. 8 showing a bioprosthetic device 510 similar to bioprosthetic device 410, described above. Bioprosthetic device 510 includes an SIS portion 512 and a synthetic portion 514 coupled to SIS portion 512. SIS portion 512 includes a top tissue layer 516 which is circular in shape and a bottom tissue layer 518 which is also circular in shape. Each of the top and bottom tissue layers 516, 518 include one or more layers of SIS tissue. Top and bottom tissue layers 516, 518 each have a diameter, D1. The synthetic portion 514 of bioprosthetic device 510 includes a mesh member 520 coupled to and positioned to lie between top and bottom tissue layers 516, 518. Mesh member 520 is circular in shape and has a diameter, D2, which is greater than diameter, D1, of synthetic portion 512. Therefore, as shown in Fig. 8, an outer rim portion 530 of mesh member 520 is provided. Similar to outer wing portions 430 of bioprosthetic device 410, shown in Fig. 7, outer rim portion 530 of bioprosthetic device 510 provides additional material for anchoring bioprosthetic device 510 to the surrounding soft tissue during surgery.

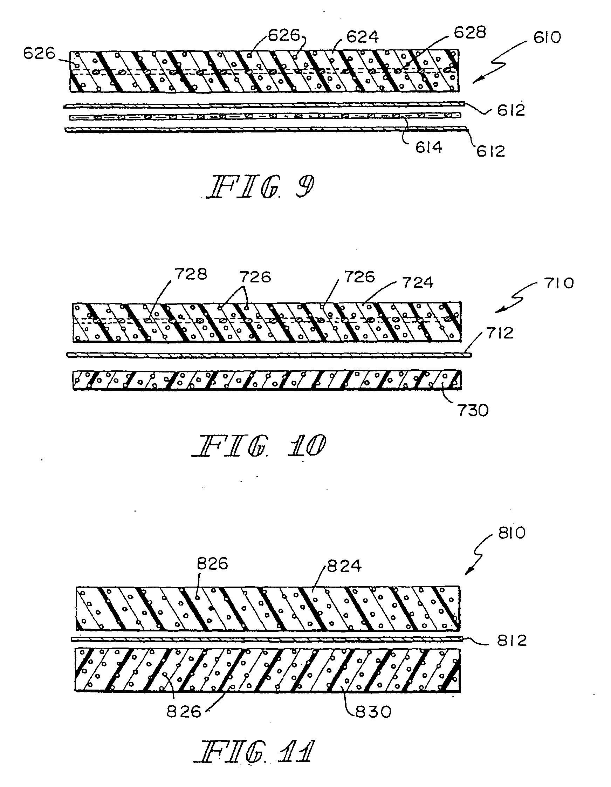

Fig. 9 shows a three-dimensional prosthetic device 610, having several SIS layers 612, a synthetic reinforcing material 614 positioned to lie between the SIS layers 612, and a three-dimensional synthetic portion 624. The SIS layer 612 may comprise any number of tissue layers. Furthermore, illustratively, if more than one layer is used, the layers may be laminated together. The device might have perforated tissue layers or any other physical configuration of SIS. As with the embodiments shown in Figs. 5 to 8, any number of SIS and reinforcing layers may be used, depending on the application.

Synthetic reinforcing material 614 illustratively comprises a two-dimensional fibrous matrix construct, as shown in Figs. 5-8, and may have the same length and width as the SIS layer, as shown in Fig. 5, may be slightly smaller, or may extend beyond the ends of the SIS layer, as shown in Figs. 7-8. Alternatively, synthetic reinforcing material may comprise a three-dimensional mesh, textile, felt, or other fibrous non-woven construct, which may be shaped or formed for the particular application. The fibres comprise any biocompatible material, including PLA, PGA, PCL, PDO, TMC, PVA, or copolymers or blends thereof. In one example, mesh material is a 95:5 copolymer of PLA/PGA.

Three-dimensional synthetic portion 624 is a non-woven material prepared to have numerous interconnecting pores or voids 626. Illustratively, the size of the voids may range from 20 to 400 microns. However, the size of the voids may be adjusted depending on the application, and the size may be manipulated by changing process steps during construction by altering freezing temperature, rate of temperature change and vacuum profile. Examples of various polymers that may be used for the foam, as well as various lyophilization profiles to control porosity, are described in US-6333029 and US-6355699. Optionally, three-dimensional synthetic portion 624 further comprises a synthetic reinforcing layer 628 embedded within the foam. Reinforcing layer 628 illustratively provides enhanced mechanical integrity to the three-dimensional synthetic portion. In an illustrated embodiment, a knitted mesh (especially made from the material sold under the trade mark Vicryl) is used. However, other reinforcing layers may be used.

Optionally, three-dimensional synthetic portion 624 may be a hybrid ECM/synthetic foam portion. In making such a foam, the polymer solution is mixed with a slurry of comminuted SIS prior to lyophilization, for example as disclosed in EP-A-1425024.

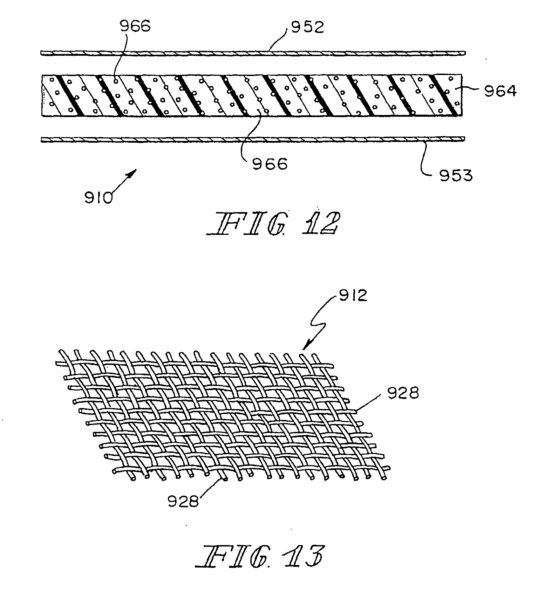

Fig. 10 shows a bioprosthetic device 710 that is similar to that of Fig. 9. In Fig. 10, the SIS layer 712 is sandwiched between two three-dimensional synthetic portions 724, 730. Illustratively, both three-dimensional synthetic portions are foams, having voids 726. As shown, three-dimensional synthetic portion 724 has a reinforcing mesh 728, while three-dimensional synthetic portion 730 does not have a reinforcing member. However, other arrangements are possible, and Fig. 11 shows an embodiment 810 where the SIS layer 812 is sandwiched between two three-dimensional synthetic portions 824, 830, neither of which has reinforcing members.

Fig. 12 shows another embodiment 910, wherein a single three-dimensional synthetic portion 964 is sandwiched between two SIS layers 952, 953. As shown, three-dimensional synthetic portion 964 is a foam, with voids 966, but other three-dimensional synthetic portions may be used.

Fig. 13 shows a woven mesh 912 made from strips 928 of SIS. Fresh, lyophilized, or laminated strips of SIS may be cut into narrower strips and woven into a mesh. The strips may be of any width, depending on the application, for example 0.1 to 20 mm, more particularly 1.0 mm wide strips. Optionally, the woven strips may be laminated together to provide enhanced mechanical support. The SIS woven mesh may be used as the SIS layer in any of the above embodiments. When used with the synthetic foams, if sufficient space is provided in the weaving, the foams will form through the spaces within the mesh.

Reinforced SIS devices may also be fabricated using other processes. For example, a synthetic polymer mesh coated with comminuted SIS (or other ECM) may be sandwiched in the middle of twenty strips of SIS (10 layers on each side), laminated under high pressure, and subsequently dried under vacuum pressure in a flat-bed gel drier system. The comminuted SIS (or other comminuted ECM) may be prepared in the manner described in US-A-2003/0044444.

Such a laminated and dried implant is significantly more resistant to delamination (175 minutes to delaminate using a water bath delamination protocol described below) as compared to implants made either with high pressure lamination but without the comminuted SIS coating (60 minutes to delaminate) or with the comminuted SIS coating but without the high pressure lamination (20 to 30 minutes to delaminate). It is believed that coating the synthetic mesh with comminuted SIS and then initiating lamination under high pressure has a synergistic effect on the resistance to delamination.

Such relatively high, positive pressure may be applied in a number of different manners. In an exemplary implementation, the relatively high, positive pressure is applied by use of a pneumatic cylinder press assembly. Other positive pressure sources may also be used. Moreover, the pressure may be applied in a wide range of magnitudes.

Implants fabricated in such a manner may have higher, and perhaps significantly higher, mechanical properties. In specific exemplary uses, such implants may be used where diseased/damaged tissue needs to be regenerated under high load conditions. For example, such implants may be used for the augmentation of damaged/resected hip capsule following primary or revision hip surgery, for patellar tendon regeneration, for the repair of large rotator cuff tears, for spinal ligament regeneration, and the like.

Other methods for fabricating reinforced SIS implants are also contemplated. For example, the surface of the synthetic polymer is characteristically hydrophobic in nature, while the SIS surface is hydrophilic. The surface of the synthetic polymer component may be modified to render it more hydrophilic, and, as a result, more compatible with the SIS surface. The more hydrophilic polymer surface creates a like-like attraction (e.g., weak force and hydrogen bonding) between the synthetic polymer component and the SIS thereby reducing the occurrences of delamination of the device. Such modification of the surface of the polymer component may also be used in conjunction with concepts described above for fabricating a pressure-laminated and vacuum-dehydrated composite.

Surface modification of the synthetic polymer component, such as a resorbable polyester, may be accomplished by numerous techniques such as, for example, traditional wet chemistry or gas plasma processing. Traditional chemistries may include surface hydrolysis and amidation techniques. Base or acid catalysed hydrolysis of the synthetic polymer (e.g., polyester) creates pendant hydroxyl and carboxylic acid moieties, while treatment with a bifunctional amine affords free amine functionalities coupled to the surface. It should be appreciated that such a bifunctional amine may have an amine on both ends thereof, or, alternatively, may have an amine on one end with any type of hydrophilic group on the other end.

Gas plasma treatment of the synthetic polymer generates high energy reactive species that bond to surfaces. For example, treatment of a polymer surface with ammonia plasma generates an amine functionalised surface. Similarly, an oxidative plasma may be produced by filtering aqueous hydrogen peroxide into the plasma chamber at approximately 400 mTorr and applying an approximately 200 Watt radio frequency for approximately 3, 5, or 10 minutes.

It should be appreciated that such treatment of the synthetic polymer may also be used to functionalise non-absorbable polymers.

By taking these approaches to strengthen SIS laminates, crosslinking of the SIS material may be avoided, thus retaining more of its biochemical and biological properties. However, to fit the needs of a given implant design, crosslinking of the SIS material may be used in conjunction with the herein described strengthening techniques.

The composite implants described herein may be used where diseased or damaged tissue needs to be regenerated under high load conditions, for example, for the augmentation of damaged/resected hip capsule following primary or revision hip surgery, for patellar or Achilles tendon regeneration, for the repair of large rotator cuff tears, for spinal ligament regeneration, etcetera.

It should be appreciated that devices may be fabricated which include a combination of both surface treatment and coating of the synthetic polymer component. For example, the synthetic polymer component may first be treated to enhance the hydrophilicity of it surface (e.g., by use of wet chemistry or gas plasma treatment). Once treated, the synthetic polymer component may be coated in comminuted SIS (or other naturally occurring extracellular matrix material) in the manner described above. Thereafter, the synthetic polymer component may be secured to layers of SIS (or other ECM). For example, the treated and coated synthetic polymer layer may be laminated to one or more SIS layers under high pressure and subsequently dried under vacuum pressure in the manner described above.

In Figs. 10 to 11, an SIS layer is sandwiched between two three-dimensional foam sections, with or without a reinforcing material embedded within the foam. Additional reinforcing layers, as shown in Fig. 9 may be used with these embodiments. Similarly, when a single three-dimensional foam portion is sandwiched between two SIS layers, as in Fig. 12, a layer of reinforcing material may be used, depending upon the application. In still another embodiment, the reinforcing portion may comprise a three-dimensional mesh or textile, and the three-dimensional foam portion may be omitted. The SIS portion might be provided as sheets, perforated sheets, or any other physical configuration of SIS. Furthermore, the synthetic portion may comprise Prolene™ (Ethicon, Inc, Somerville, NJ) meshes and/or sutures, Vicryl™ (Ethicon, Inc, Somerville, NJ) meshes and/or sutures, Mersilene™ (Ethicon, Inc, Somerville, NJ) meshes, PDS II™ (Ethicon, Inc., Somerville, NJ) meshes or sutures, Panacryl™ (Ethicon, Inc., Somerville, NJ) meshes or sutures, and Monocryl™ meshes or sutures, for example. Additional two or three-dimensional meshes may be constructed for particular applications. The invention also provides bioprosthetic devices where the SIS portion includes any number of tissue layers and where multiple tissue layers are positioned to lie along each synthetic layer. The SIS layers may be dehydrated prior to or subsequent to assembly of the device.

### Example 1

Sheets of clean, disinfected porcine SIS material were obtained as described in US-4902508 and US-4956178. Ten strips, 89 mm (3.5 inch) wide and 152 mm (6 inch) long were cut. The strips were hydrated by placing in RO water, at room temperature, for 5 minutes.

To assemble the implant, five SIS strips were placed on top of each other, while ensuring no air bubbles were trapped between the strips. A knitted mesh made of the material sold under the trade mark Panacryl , 51 mm (2 inch) wide and 127 mm (5 inch) long, was placed centrally on the 5-layer thick SIS strip. The mesh had been pretreated to remove any traces of oil or other contaminants due to handling. This was done by a series of rinses, each 2 minutes long, in 100%, 90%, 80%, 70% ethanol (200 proof) in RO water, followed by a final 5 minute in RO water. Subsequently, a second 5-layer thick strip of SIS was assembled and placed to sandwich the mesh between the two SIS strips.

The implant was dried under vacuum pressure using a gel drier system (Model FB-GD-45, Fisher Scientific, Pittsburgh, PA) for 3 hours. The gel drier bed temperature was set at 30°C for the procedure. This drying procedure results in "squeezing out" of the bulk water in the implant and also reduces the amount of bound water within the tissue, resulting in a final moisture of between 7%-8%. This process also results in a physical crosslinking between the laminates of SIS and between the mesh and adjacent SIS laminates.

Non-reinforced SIS strips were made in the same way as described, except that no mesh material was placed between the strips of SIS.

### Example 2

This example describes the preparation of three-dimensional composite tissue implants incorporating a biodegradable SIS laminated sheet, a synthetic reinforcement in the form of a biodegradable mesh, and a synthetic degradable foam.

A solution of the polymer to be lyophilized to form the foam component was prepared in a four step process. A 95:5 weight ratio solution of 1,4-dioxane/(40/60 PCL/PLA) was made and poured into a flask. The flask was placed in a water bath, stirring at 60-70°C for 5 hrs. The solution was filtered using an extraction thimble, extra coarse porosity, type ASTM 170-220 (EC) and stored in flasks.

A three-dimensional mesh material composed of a 95:5 copolymer of polylactic/polyglycolic acid (PLA/PGA) knitted mesh was rendered flat to control curling by using a compression moulder at 80°C for 2 min. After preparing the mesh, 0.8 mm metal shims were placed at each end of a 102 mm (4 inch) square aluminum mould, and the mesh was sized to fit the mould. The synthetic mesh was then laid into the mould, covering both shims. Next, an SIS laminated sheet was placed over the mesh followed by additional shims to cover the edges of the SIS and synthetic mesh.

The polymer solution (40:60 PCL/PLA) was added into mould such that the solution covered the sheet of SIS as well as the mesh and reached a level of 3.0 mm in the mould.

The mould assembly then was placed on the shelf of the lyophilizer (Virtis, Gardiner, NY) and the freeze dry sequence begun. The freeze dry sequence used in this example was: 1) -17°C for 60 minutes; 2) -5°C for 60 minutes under vacuum of 100 mT; 3) 5°C for 60 minutes under vacuum of 20 mT; 4) 20°C for 60 minutes under vacuum of 20 mT.

After the cycle was completed, the mould assembly was taken out of the freeze drier and allowed to degas in a vacuum hood for 2 to 3 hours, and stored under nitrogen.

The resultant bioprosthetic device has a structure as illustrated in Fig. 9. The three-dimensional mesh provides both mechanical strength and three-dimensional structure to the resultant device. The foam may be shaped or sculpted for the particular application, and the mesh/SIS layers may be trimmed to fit. It is also understood that the mould could be provided in the desired shape, reducing or obviating the need for sculpting or trimming.

### Example 3

This example uses the process outlined in Example 2 to fabricate a biodegradable composite scaffold of the present invention where the foam component is a 65:35 PGA/PCL copolymer.

### Example 4

This example uses the process outlined in Example 2 to fabricate a biodegradable composite scaffold of the present invention where the synthetic knitted mesh component is composed of 100% PDO.

### Example 5

This example uses the process outlined in Example 2 to fabricate a biodegradable composite scaffold of the present invention where in place of a three-dimensional mesh, the synthetic component is a non-woven fibrous structure composed of either 100% PDO, 100% 90/10 PGA/PLA or a combination of the two.

### Example 6

This example uses the process outlined in Example 2 to fabricate a biodegradable composite scaffold of the present invention where the SIS component is soaked overnight in the polymer solution (5% wt 60/40 PLA/PCL in dioxane) prior to placement over the synthetic mesh. Enhanced lamination between the components was found when this additional soaking step was added to the process as evidenced by a composite with a greater degree of handlability.

### Example 7

This example uses the process outlined in Example 2 to fabricate a biodegradable composite scaffold of the present invention where the SIS component is a single layer sheet rather than a laminated sheet.

### Example 8

This example uses the process outlined in Example 2 to fabricate a biodegradable composite scaffold of the present invention where the SIS laminated sheet is perforated with holes ranging from 1 mm to 1 cm. These perforations allow for enhanced penetration of the polymer solution through the SIS sheet.

### Example 9

This example uses the process outlined in Example 2 to fabricate a biodegradable composite scaffold of the present invention where the SIS reinforcing component is a "woven mesh" of laminated strips sandwiched between two layers of 60/40 PLA/PCL foam. Fig. 13 shows such a woven mesh. Fig. 11, wherein the SIS layer is a woven mesh of Fig. 13, illustrates the construct of this Example.

### Example 10

A soaking test was performed to test resistance to delamination. Implants made as specified in Example 1 (both reinforced and non-reinforced) were cut into several strips 1 cm wide by 5 cm long, using a #10 scalpel blade. The strips were immersed in RO water, at room temperature for 1, 2, 5, 10, 20, 30, or 60 minutes. Delamination was detected at the edges of the implants by direct visual observation. All implants showed obvious signs of delamination at 1 hour. In non-reinforced implants, delamination was first visually observed between 40 to 60 minutes, whereas in the reinforced samples delamination was apparent between 20 to 30 minutes.

### Example 11

This example illustrates the enhanced mechanical properties of a construct reinforced with absorbable mesh. Preparation of three-dimensional elastomeric tissue implants with and without a reinforcement in the form of a biodegradable mesh are described. While a foam is used for the elastomeric tissue in this example, it is expected that similar results will be achieved with an ECM and a biodegradable mesh.

A solution of the polymer to be lyophilized to form the foam component was prepared in a four step process. A 95/5 weight ratio solution of 1,4-dioxane/(40/60 PCL/PLA) was made and poured into a flask. The flask was placed in a water bath, stirring at 70°C for 5 hrs. The solution was filtered using an extraction thimble, extra coarse porosity, type ASTM 170-220 (EC) and stored in flasks.

Reinforcing mesh materials formed of a 90/10 copolymer of polyglycolic/polylactic acid (PGA/PLA) knitted (Code VKM-M) and woven (Code VWM-M), both sold under the trade mark VICRYL were rendered flat by ironing, using a compression moulder at 80°C/2 min. After preparing the meshes, 0.8 mm shims were placed at each end of a 153 mm square aluminium mould, and the mesh was sized (14.2 mm) to fit the mould. The mesh was then laid into the mould, covering both shims. A clamping block was then placed on the top of the mesh and the shim such that the block was clamped properly to ensure that the mesh had a uniform height in the mould. Another clamping block was then placed at the other end, slightly stretching the mesh to keep it even and flat.

As the polymer solution was added to the mould, the mould was tilted to about a 5 degree angle so that one of the non-clamping sides was higher than the other. Approximately 60 ml of the polymer solution was slowly transferred into the mould, ensuring that the solution was well dispersed in the mould. The mould was then placed on a shelf in a Virtis (Gardiner, NY), Freeze Mobile G freeze dryer. The following freeze drying sequence was used: 1) 20°C for 15 minutes; 2) -5°C for 120 minutes; 3) -5°C for 90 minutes under vacuum 100 milliTorr; 4) 5°C for 90 minutes under vacuum 100 milliTorr; 5) 20°C for 90 minutes under vacuum 100 milliTorr. The mould assembly was then removed from the freezer and placed in a nitrogen box overnight. Following the completion of this process the resulting implant was carefully peeled out of the mould in the form of a foam/mesh sheet.

Non-reinforced foams were also fabricated. To obtain non-reinforced foams, however, the steps regarding the insertion of the mesh into the mould were not performed. The lyophilization steps above were followed.

### Example 12

Lyophilized 40/60 polycaprolactone/polylactic acid, (PCL/PLA) foam, as well as the same foam reinforced with an embedded VICRYL knitted mesh, were fabricated as described in Example 3. These reinforced implants were tested for suture pull-out strength and compared to non-reinforced foam prepared following the procedure of Example 11.

For the suture pull-out strength test, the dimensions of the specimens were approximately 5 cm X 9 cm. Specimens were tested for pull-out strength in the wale direction of the mesh (knitting machine axis). A size 0 polypropylene monofilament suture (Code 8834H), sold under the trade mark PROLENE (by Ethicon, Inc., Somerville, NJ) was passed through the mesh 6.25 mm from the edge of the specimens. The ends of the suture were clamped into the upper jaw and the mesh or the reinforced foam was clamped into the lower jaw of an Instron model 4501 (Canton, MA). The Instron machine, with a 9.1 kg (20 lb) load cell, was activated using a cross-head speed of 2.54 cm per minute. The ends of the suture were pulled at a constant rate until failure occurred. The peak load experienced during the pulling was recorded.

The results of this test are shown below in the table in the following Table.

| Time | Foam | Mesh | Foamed Mesh |
|---|---|---|---|
| 0 Day | 0.21 kg (0.46 lbs) | 2.41 ± 0.36 kg (5.3 ± 0.8 lbs) | 2.59 ±0.14 kg (5.7 ± 0.3 lbs) |
| 7 Day | - | 1.82 ± 0.45 kg 4.0 ± 1.0 lbs | 2.27 ± 0.23 kg (5.0 ± 0.5 lbs) |

Note that the 7 day measurements involved exposure to phosphate buffered saline at 37°C in a temperature controlled water bath.

These data show that a reinforced foam has improved pull-out strength verses either foam or mesh alone.

### Example 13

Sheets of clean, disinfected porcine SIS material were obtained as described in US-4902508 and US-4956178. Twenty strips, 3.5 inches wide and 6 inches long were cut. The strips were hydrated by placing in RO water, at room temperature, for 5 minutes.

To assemble the implant, ten SIS strips were placed longitudinally on top of each other, while ensuring no air bubbles were trapped between the strips. A knitted mesh made of the material sold under the trade mark Panacryl , 51 mm (2 inch) wide and 127 mm (5 inch) long, was immersed in a comminuted SIS suspension (disclosed in US-A-2003/004444) (approximately 1% solids w/v). This results in a near-uniform coating of the synthetic mesh with the wet fibres of the SIS suspension such that the SIS fibres are intertwined and interlocked with the porous knitted mesh. The coated mesh was placed centrally on the 10-layer thick SIS strip. Subsequently, a second 10-layer thick strip of SIS was assembled and placed to sandwich the coated mesh between the two SIS strips.

Lamination of the thus assembled implant was initiated under high pressure using a pneumatic cylinder press (Model BTP-501-A, TRD Manufacturing Inc., Loves Park, IL 61111.) The press was operated at 275 kPa (40 psi) air pressure to drive the piston, which resulted in a total compressive force of approximately 1814 kg (4000 lb) on the assembled implant. This force created an approximate average lamination pressure of 1241 kPa (180 psi) on the implant. The sample was compressed for 15 minutes at room temperature. This process resulted in a "squeezing out" of most of the bulk water associated with the SIS laminates and comminuted SIS and created a partially wet laminated implant.

The implant was subsequently dried under vacuum pressure using a flat-bed gel drier system (Model FB-GD-45, Fisher Scientific, Pittsburgh, PA) for 3 hours. The gel drier bed temperature was set at 30°C for the procedure. This drying procedure resulted in a further reduction of the bulk water associated with the implant and also reduced the amount of bound water within the implant, resulting in a final moisture content between 7%-8%. This process also results in a physical crosslinking between the laminates of SIS and the comminuted SIS coating the synthetic mesh by further increasing the surface contact area of SIS material.

Implants were also made as described above but without coating the mesh with the comminuted SIS fibres.

### Example 14

An agitation test was performed to test for resistance to delamination. Highpressure laminated implants made as described in Example 13 (both with and without SIS coating on the mesh) were cut into several strips 1 cm wide and 5 cm long. Each strip was placed in 20 mL of reverse osmosis water at room temperature in a 50 mL glass flask. The flasks were secured on a shaker table set to agitate the samples at 300 rpm. Every five minutes the strips were examined for delamination between the SIS laminates and the synthetic mesh. On average, reinforced implants without the comminuted SIS-coated mesh delaminated after 60 minutes of agitation, whereas, reinforced implants with the comminuted SIS-coating delaminated after 175 minutes.

It is expected that high pressure laminated SIS implants reinforced with a comminuted SIS coated synthetic mesh will also have higher (and perhaps significantly higher) mechanical properties (e.g. higher ball burst strength) as compared with implants made without high pressure lamination or without a comminuted SIS coating on the synthetic mesh.

## Claims

1. A method of making a bioprosthetic device, the method comprising the steps of:
coating a synthetic layer with a comminuted naturally occurring extracellular matrix material,
positioning the coated synthetic layer between a first layer of naturally occurring extracellular matrix material and a second layer of naturally occurring extracellular matrix material to make an assembly, and
applying positive pressure to the assembly.

2. The method of claim 1, wherein the applying step comprises pressing the assembly together.

3. A method of making a bioprosthetic device, the method comprising the steps of:
coating a synthetic layer with comminuted naturally occurring extracellular matrix material,
positioning the coated synthetic layer between a first layer of naturally occurring extracellular matrix material and a second layer of naturally occurring extracellular matrix material to make an assembly, and
operating a press to exert positive pressure on the assembly.

4. The method of claim 1 or claim 3, wherein the applying step comprises pressing the assembly with a pneumatic press.

5. The method of claim 1, wherein the applying step comprises:
positioning the assembly in a press, and
operating the press to exert pressure on assembly.

6. The method of claim 1 or claim 3, further comprising the step of drying the assembly after the applying step.

7. The method of claim 6, wherein the drying step comprises drying the assembly under vacuum pressure.

8. The method of claim 1 or claim 3, wherein both the first and second naturally occurring extracellular matrix material layers comprise an SIS layer.

9. The method of claim 8, wherein the SIS layer comprises a plurality of SIS strips laminated together.

10. The method of claim 8, wherein the SIS layer comprises a woven mesh of strips of SIS.

11. The method of claim 1 or claim 3, wherein the synthetic layer comprises a fibrous material.

12. The method of claim 11, wherein the fibrous material is selected from the group consisting of mesh, textile, and felt.

13. The method of claim 11, wherein the fibrous material is a bioabsorbable material selected from the group consisting of PLA, PGA, PCL, PDO, TMC, PVA, copolymers thereof, and blends thereof.

14. The method of claim 1 or claim 3, wherein the coating step comprises immersing the synthetic layer in a suspension of comminuted naturally occurring extracellular matrix material.
